Europäisches Patentamt

European Patent Office

Office européen des brevets

(19)

(11) Veröffentlichungsnummer: **0 089 946**
A1

(12) EUROPÄISCHE PATENTANMELDUNG

(21) Anmeldenummer: 83890037.1

(22) Anmeldetag: 15.03.83

(51) Int. Cl.³: **G 01 N 27/22**
**G 01 R 27/26**

(30) Priorität: 18.03.82 AT 1086/82

(43) Veröffentlichungstag der Anmeldung:
28.09.83 Patentblatt 83/39

(84) Benannte Vertragsstaaten:
DE FR GB IT

(71) Anmelder: AVL GESELLSCHAFT FÜR
VERBRENNUNGSKRAFTMASCHINEN UND
MESSTECHNIK MBH.PROF.DR.DR.H.C.HANS LIST
Kleiststrasse 48
A-8020 Graz(AT)

(72) Erfinder: Faschingleitner, Leopold
Anzenbach 5
A-3240 Mank(AT)

(72) Erfinder: Krempl, Peter
Leonhardstrasse 100/2/7
A-8010 Graz(AT)

(72) Erfinder: Ruckenbauer, Friedrich
Lessingstrasse 5
A-8010 Graz(AT)

(74) Vertreter: Krause, Walter, Dr. Dipl.-Ing.
Margaretenstrasse 21
A-1040 Wien(AT)

(54) Einrichtung zur Bestimmung des Mischungsverhältnisses von Flüssigkeiten.

(57) Die Kapazität eines von einem Gemisch von Flüssigkeiten mit unterschieldlichen Dielektrizitätskonstanten erfüllten Meßkondensators wird bestimmt und dem gesuchten Mischungsverhältnis der Flüssigkeiten zugeordnet. Zur Kapazitätsbestimmung dient eine kapazitive Meßbrücke, die neben dem mit dem Gemisch erfüllten Meßkondensator (Cin) einen thermisch mit diesem gekoppelten Bezugskondensator (Ct) umfaßt, dessen Dielektrikum eine dem zu untersuchenden Gemisch annähernd gleiche Temperaturabhängigkeit der Dielektrizitätskonstante aufweist.

FIG.1

## Einrichtung zur Bestimmung des Mischungsverhältnisses von Flüssigkeiten

Die Erfindung betrifft eine Einrichtung zur Bestimmung des Mischungsverhältnisses von Flüssigkeiten mit unterschiedlichen Dielektrizitätskonstanten, insbesonders von Benzin-Methanol oder Benzin-Äthanol, wobei die Dielektrizitätskonstante des Gemisches durch Messung der Kapazität eines von dem Gemisch erfüllten Meßkondensators bestimmt und der gesuchten Konzentration zugeordnet wird.

Es sind Einrichtungen und Verfahren zur Bestimmung des Mischungsverhältnisses von Flüssigkeiten bekannt, welche durch eine für das Gemisch bzw. dessen Transport praktisch rückwirkungsfreie Messung von Größen wie beispielsweise dem Brechungsindex, der Schallgeschwindigkeit, der Leitfähigkeit oder auch der Dielektrizitätskonstante in vorteilhafter Weise neben der Bestimmung des Mischungsverhältnisses von Einzelproben auch eine kontinuierliche Bestimmung des Mischungsverhältnisses erlauben. Allen bekannten derartigen Verfahren und Einrichtungen haftet jedoch der Nachteil einer sehr großen Temperaturabhängigkeit an. Es besteht zwar prinzipiell die Möglichkeit, die Temperaturabhängigkeit der zu messenden Größe bzw. direkt des Mischungsverhältnisses empirisch zu bestimmen und die sodann gemessenen Werte rechnerisch oder beispielsweise unmittelbar über eine der eigentlichen Messung nachgeschaltete Mikroelektronik oder dgl. zu kompensieren. Abgesehen von dem erhöhten Aufwand für eine derartige Korrektur sind auch zusätzliche Thermosensoren zur Bestimmung der tatsächlich vorherrschenden Temperatur erforderlich.

Aufgabe der vorliegenden Erfindung ist es, die genannten Nachteile der bekannten Einrichtungen zur Mischungsverhältnisbestimmung von Flüssigkeiten zu vermeiden und insbesonders eine Einrichtung der eingangs genannten Art dahingehend zu verbessern, daß auf einfache Weise eine Kompensation der Auswirkungen von Temperaturschwankungen des Gemisches möglich ist.

Dies wird gemäß der Erfindung dadurch erreicht, daß zur Messung der Kapazität in an sich bekannter Weise eine kapazitive Meßbrücke mit einem Meßkondensator und einem mit diesem elektrisch verbundenen Bezugskondensator vorgesehen und der letztere mit einem Dielektrikum gefüllt ist, das eine dem zu untersuchenden Gemisch zumindest annähernd gleiche Temperaturabhängigkeit der Dielektrizitätskonstante aufweist und daß der Meßkondensator und der Bezugskondensator miteinander in thermisch leitender Verbindung stehen. Da die Brückenspannung der kapazitiven Meßbrücke nur vom vorliegenden Kapazitätsverhältnis abhängt, ist - vorausgesetzt, daß beide Kapazitäten bzw. das in ihnen befindliche Dielektrikum die gleiche Temperaturabhängigkeit aufweisen - die Brückenspannung unabhängig von der Temperatur. Die obigen Ausführungen gelten natürlich sinngemäß auch für einen der kapazitiven Meßbrücke äquivalenten kapazitiven Spannungsteiler, bei dem die Teilerspannung - soferne beide Kondensatoren die gleiche Temperaturabhängigkeit ihrer Kapazität aufweisen - unabhängig von der Temperatur ist. Aus der gemessenen Brückenspannung bzw. Teilerspannung ist auf einfache und bekannte Weise die Dielektrizitätskonstante und daraus das Mischungsverhältnis bestimmbar.

In weiterer Ausgestaltung der Erfindung ist vorgesehen, daß der Meßkondensator und der Bezugskondensator als konzentrisch ineinander geschaltete Zylinderkondensatoren ausgebildet sind. Dadurch ist auf sehr einfache Weise eine gute thermische Koppelung zwischen den beiden zugehörigen Kondensatoren erreicht, wodurch eine Temperaturunabhängigkeit der Messung gewährleistet ist.

In diesem Zusammenhang ist es besonders vorteilhaft, wenn in weiterer Ausgestaltung der Erfindung der Meßkondensator innerhalb des Bezugskondensators angeordnet ist und wenn die innere Elektrode des Meßkondensators als Rohr ausgebildet ist, das durch eine senkrecht zu seiner Längsachse stehende Trennwand unterteilt ist und zu beiden Seiten der Trennwand Bohrungen aufweist, die das Zu- und Abfließen des zu messenden Gemisches in den Meßkondensator ermöglichen. Diese Ausbildung ist konstruktiv besonders einfach und kompakt, wobei die gute thermische Koppelung der beiden ineinander geschachtelten Kondensatoren trotzdem erhalten bleibt.

Die Erfindung wird im folgenden anhand der in der Zeichnung dargestellten Ausführungsbeispiele näher erläutert.

Fig. 1 zeigt ein Prinzipschaltbild einer Einrichtung gemäß der vorliegenden Erfindung,

Fig. 2 zeigt ein Beispiel für die in einer Einrichtung nach der vorliegenden Erfindung vorgesehene thermische Koppelung der Kondensatoren,

Fig. 3 zeigt ein Detail einer anderen Ausführungsform nach der vorliegenden Erfindung,

Fig. 4 zeigt einen Schnitt entlang der Linie IV-IV in Fig. 3, und

Fig. 5 zeigt einen Auschnitt aus einem weiteren Ausführungsbeispiel der Erfindung.

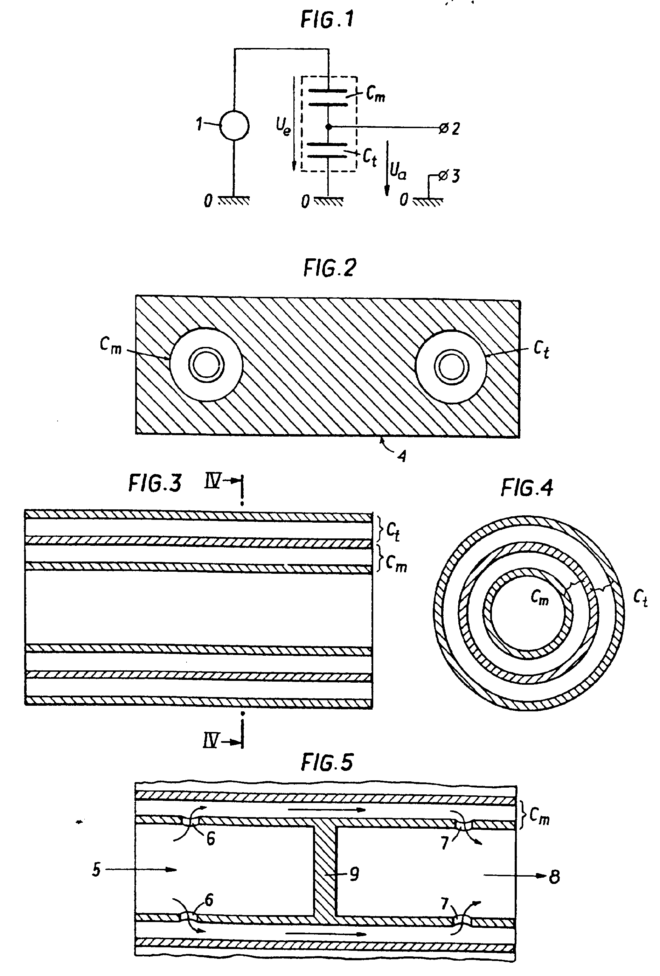

In der in Fig. 1 schematisch dargestellten Einrichtung ist der Meßkondensator, dessen Kapazität zur Bestimmung der Dielektrizitätskonstante eines auf nicht näher dargestellte Weise zwischen seine Elektroden einbringbaren Flüssigkeitsgemisches gemessen werden soll, mit $C_m$ und der im kapazitiven Spannungsteiler zugehörige Bezugskondensator, dessen Kapazität bekannt ist, mit $C_t$ bezeichnet. Über eine Spannungsquelle 1 ist eine Spannung $U_e$ an den Spannungsteiler gelegt; die Massekontakte sind mit 0 bezeichnet.

Die für die Bestimmung der unbekannten Kapazität $C_m$ zu

messende Spannung $U_a$ wird an einem Punkt zwischen den beiden Kapazitäten $C_m$ und $C_t$ abgegriffen und steht an den Kontakten 2 und 3 zur Verfügung.

Aus der aus der Elektrotechnik bekannten Beziehung

$$U_a = U_e \frac{C_m}{C_m + C_t}$$

kann die Kapazität des Meßkondensators berechnet werden. Aus dieser Kapazität kann weiters auf sehr einfache Weise die Dielektrizitätskonstante und daraus über eine einmal aufgenommene Eichkurve das gesuchte Mischungsverhältnis einer zwischen den Elektroden des Meßkondensators einbringbaren Flüssigkeitsmischung bestimmt werden. Die Eichkurve kann natürlich auch unmittelbar für die Teilerspannung $U_a$ aufgestellt werden, womit die vorherige Berechnung der Kapazität des Meßkondensators entfällt.

Um die relativ starke Temperaturabhängigkeit der Dielektrizitätskonstante auf einfache Weise zu kompensieren, sind der Meßkondensator $C_m$ und der zugehörige Bezugskondensator $C_t$ - wie strichliert angedeutet - thermisch gekoppelt und befinden sich somit auf gleicher Temperatur. Außerdem ist zwischen den Elektroden des Bezugskondensators $C_t$ auf hier nicht dargestellte Weise ein Dielektrikum eingebracht, welches eine dem im Meßkondensator $C_m$ befindlichen zu untersuchenden Gemisch zumindest annähernd gleiche Temperaturabhängigkeit seiner Dielektrizitätskonstante aufweist.

Diese thermische Koppelung der beiden Kondensatoren $C_m$ und $C_t$ kann beispielsweise auf die in Fig. 2 dargestellte Art erfolgen, wo die äußere Elektrode der als Zylinderkondensator ausgebildeten beiden Kondensatoren in einem Block 4 aus gut wärmeleitendem Material, beispielsweise einem Kupferblock, angeordnet ist. Die inneren Elektroden beider Kondensatoren sind konzentrisch zu den äußeren im Kupferblock angeordnet. Durch die Sicherstellung des guten Wärmeüberganges zwischen den beiden Kondensatoren sind - bei zumindest annähernd gleicher Temperaturabhängigkeit der zwischen den jeweiligen Elektroden befindlichen Dielektrika - die Nachteile einer Temperaturabhängigkeit der Kapazität des Meßkondensators vermieden.

In den Fig. 3 und 4 ist eine andere Möglichkeit zur einfachen thermischen Koppelung des Meßkondensators $C_m$ mit dem Bezugskondensator $C_t$ dargestellt. Der Meßkondensator $C_m$ und der zugehörige Bezugskondensator $C_t$ sind als konzentrisch ineinander geschachtelte Zylinderkondensatoren ausgebildet, wodurch die äußere Elektrode des Meßkondensators gleichzeitig die innere Elektrode des Bezugskondensators darstellt und eine sichere thermische Koppelung gewährleistet ist. Die Anschlüsse zur Zuführung und Ableitung der in ihrem Mischungsverhältnis zu untersuchenden Flüssigkeit zwischen die Elektroden des Meßkondensators sind nicht dargestellt; gleiches gilt auch für das seine Dielektrizitätskonstante ausschließlich temperaturabhängig ändernde Dielektrikum zwischen den Elektroden des Bezugskondensators.

In Fig. 5 ist eine vorteilhafte Möglichkeit zur Ausbildung des in ähnlicher Weise wie in den Fig. 3 und 4 konzentrisch innerhalb des Bezugskondensators angeordneten Meßkondensators $C_m$ gleichzeitig als Zuführung und Ableitung des zu untersuchenden Flüssigkeitsgemisches dargestellt. Das entlang des Pfeiles 5 zuströmende Gemisch tritt durch Bohrungen 6 aus dem von der inneren Elektrode des Meßkondensators umschlossenen Raum in den zwischen den Elektroden befindlichen Raum über und gelangt durch Bohrungen 7 wieder in den Innenraum zurück, wobei das entlang des Pfeiles 8 abströmende Gemisch vom zuströmenden Gemisch durch eine Zwischenwand 9 getrennt ist.

Es ist auf diese Weise eine sehr einfache und temperaturunabhängige kontinuierliche Bestimmung des Mischungsverhältnisses von Flüssigkeiten mit unterschiedlichen Dielektrizitätskonstanten, wie insbesondere von Benzin-Methanol
oder Benzin-Äthanol, möglich.

Patentansprüche:

1. Einrichtung zur Bestimmung des Mischungsverhältnisses von Flüssigkeiten mit unterschiedlichen Dielektrizitätskonstanten, insbesondere von Benzin-Methanol oder Benzin-Äthanol, wobei die Dielektrizitätskonstante des Gemisches durch Messung der Kapazität eines von dem Gemisch erfüllten Meßkondensators bestimmt und der gesuchten Konzentration zugeordnet wird, d a d u r c h   g e k e n n z e i c h n e t , daß zur Messung der Kapazität in an sich bekannter Weise eine kapazitive Meßbrücke mit einem Meßkondensator ($C_m$) und einem mit diesem elektrisch verbundenen Bezugskondensator ($C_t$) vorgesehen und der letztere mit einem Dielektrikum gefüllt ist, das eine dem zu untersuchenden Gemisch zumindest annähernd gleiche Temperaturabhängigkeit der Dielektrizitätskonstante aufweist und daß der Meßkondensator ($C_m$) und der Bezugskondensator ($C_t$) miteinander in thermisch leitender Verbindung stehen.

2. Einrichtung nach Anspruch 1, dadurch gekennzeichnet, daß der Meßkondensator ($C_m$) und der Bezugskondensator ($C_t$) als konzentrisch ineinander geschachtelte Zylinderkondensatoren ausgebildet sind.

3. Einrichtung nach Anspruch 2, dadurch gekennzeichnet, daß der Meßkondensator ($C_m$) innerhalb des Bezugskondensators ($C_t$) angeordnet ist und daß die innere Elektrode des Meßkondensators ($C_m$) als Rohr ausgebildet ist, das durch eine senkrecht zu seiner Längsachse stehende Trennwand (9) unterteilt ist und zu beiden Seiten der Trennwand Bohrungen (6, 7) aufweist, die das Zu- und Abfließen des zu messenden Gemisches in den Meßkondensator ($C_m$) ermöglichen (Fig. 5)

1983 02 09
Kr/Fe

0089946

**FIG.1**

**FIG.2**

**FIG.3**

**FIG.4**

**FIG.5**

### EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-3 816 811 (R.M.H. CMELIK K.) <br> * Ansprüche 1-9; Figuren 2, 3; Spalte 3, Zeilen 40-57 * | 1,2 | G 01 N 27/22 <br> G 01 R 27/26 |
| | --- | | |
| Y | US-A-3 903 478 (D.E. STUART et al.) <br> * Anspruch 1 * | 1,2 | |
| | --- | | |
| Y | US-A-2 599 583 (R.A. ROBINSON et al.) <br> * Figur 1; Spalte 5, Zeilen 21-39; Ansprüche 1-12 * | 1,2 | |
| | --- | | |
| Y | US-A-2 485 579 (M.A. ELLIOTT) <br> * Spalte 3, Zeilen 29-34 * | 1 | |
| | --- | | RECHERCHIERTE SACHGEBIETE (Int. Cl. 3) |
| Y | GB-A-1 287 748 (SHELL INTERNATIONALE RESEARCH MAATSCHAPPIJ N.V.) <br> * Seite 4, Zeilen 47-61 * | 1 | G 01 N 27/00 <br> G 01 R 27/00 |
| | ---     -/- | | |

Der vorliegende Recherchenbericht wurde für alle Patentansprüche erstellt.

| Recherchenort <br> BERLIN | Abschlußdatum der Recherche <br> 20-06-1983 | Prüfer <br> DIETRICH A. |
|---|---|---|

**Europäisches Patentamt**

**EUROPÄISCHER RECHERCHENBERICHT**

00899946

Nummer der Anmeldung

EP 83 89 0037

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | REVUE DE PHYSIQUE APPLIQUEE, Tome 15, Nr. 10, Oktober 1980, Paris A. MOLITON et al. "Spectromètre de mesures diélectriques automatisé dans le domaine de température (77 K-380 K) et dans l'intervalle continu de fréqu ence (500 Hz-1MHz), Seiten 1563-1570 * Seite 1564, Spalte 2,; Seiten 1565, 1566 * | 1 | |
| | --- | | |
| A | US-A-3 916 300 (D.M. CHISDES et al.) * Ansprüche 1-3 * | 1,2 | |
| | --- | | |
| A | GB-A-2 052 749 (M. FAWZY EL-MENSCHAWY et al.) * Seite 2, Zeilen 7-14 * | 1 | |
| | ----- | | RECHERCHIERTE SACHGEBIETE (Int. Cl. ³) |

Der vorliegende Recherchenbericht wurde für alle Patentansprüche erstellt.

| Recherchenort BERLIN | Abschlußdatum der Recherche 20-06-1983 | Prüfer DIETRICH A. |
|---|---|---|